# EUROPEAN PATENT SPECIFICATION

(11) **EP 2 412 322 B1**
(45) Date of publication and mention of the grant of the patent: **17.04.2013**
(21) Application number: 11175259.8
(22) Date of filing: 25.07.2011
(51) Int. Cl.: A61B 17/16

(54) **Milling tool for prosthetic surgery operations**
Fräswerkzeug für chirurgische Protheseoperationen
Outil de fraisage pour opérations chirurgicales prothétiques

(30) Priority: 26.07.2010 IT UD20100153
(43) Date of publication of application: 01.02.2012
(73) Proprietor: HPF S.P.A., 33030 FORGARIA NEL FRIULI (UD) (IT)
(72) Inventor: Lualdi, Tommaso, 33034 Fagagna (UD) (IT)
(74) Representative: Petraz, Gilberto Luigi

(56) References cited:
- DE-A1-102005 058 107
- US-A- 4 023 572
- US-A- 5 203 653
- US-A- 5 376 092
- US-A1- 2007 142 840
- US-B1- 6 475 221

## Description

### FIELD OF THE INVENTION

The present invention concerns a milling tool for prosthetic surgery operations, used to make a bone seating, for example to install an acetabular prosthesis of the hip, or a shoulder prosthesis or other.

### BACKGROUND OF THE INVENTION

Milling tools in general are known, used during prosthetic surgery operations and conformed to achieve coordinated and mating bone seatings suitable to dispose and implant relative components of surgical prostheses.

In particular, milling tools are known used to make semi-spherical seatings, or in any case shaped like a spherical cap, suitable for the installation of coordinated acetabular cups of hip prostheses.

These known tools provide a cap body, hollow inside, having sizes correlated to the bone seating to be achieved and on which a plurality of through holes are made, provided with a cutting edge,in order to perform a mechanical action of excavating the bone. The cap body is mounted on a manual or automatic manipulator, in order to actuate the rotation thereof.

In this way, by rotating and performing a mechanical action, this type of milling tool substantially makes an impression of a desired size and conformation on the bone, substantially corresponding to the cap body.

One disadvantage of this type of known tool is that the assembly of the cap body on the relative manipulator, for various reasons for example connected to the skillfulness of the installation or the mechanical conformation of the attachment or other, may not be precise and/or stable: this influences the rotation of the cap body, which may be unbalanced, to the detriment of the excavation of the bone.

Another disadvantage of this type of known tool is that, due to their conformation, the through holes allow the tissue excavated by the cutting edges to progressively accumulate until they block them, causing possible jamming and also a loss of efficiency of the tool on the bone, so that therefore the quality of the operations performed is reduced.

The jamming of the millers usually entails an increase in the drive torque, which can cause damage to the milled tissue.

Moreover, since the individual cutting edges are quite small since they are made on the through holes, they have operating difficulty in simultaneously incising both hard tissue, like the bones, for example the necrotic bone, and soft tissues, like the ligaments, and can therefore damage the bone structure.

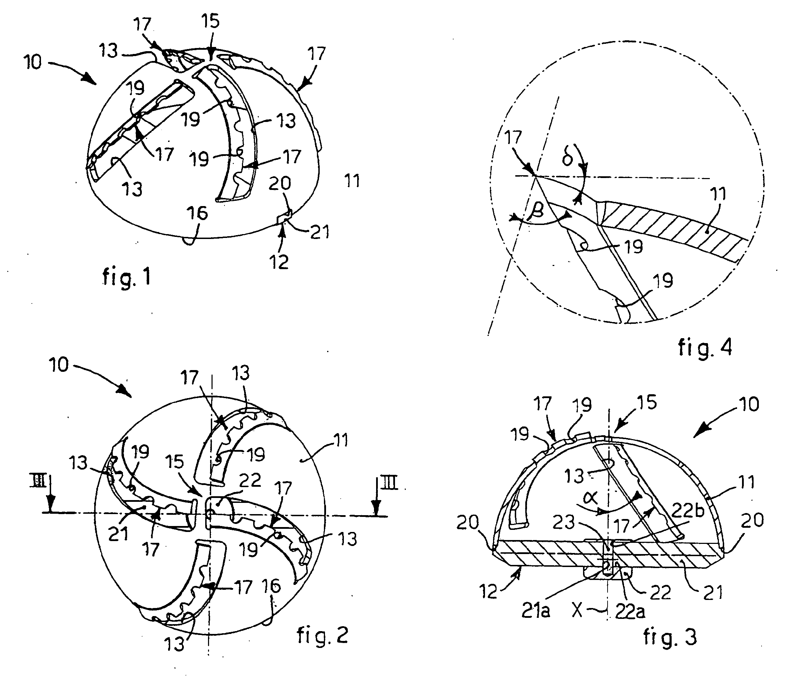

Document US-A-4,023,572 discloses a surgical milling tool including a body having a hemispherically-shaped outer surface and defining an internal cavity. A tool support is releasably connected to the tool body by means of a spring member, or a lever or a hook-shaped element. The embodiment shown in Figs. 4-6 forms the basis for the two-part form of claim 1.

Document US-B-6,475,221 discloses a surgical reamer having a hollow dome with apertures spaced apart arranged in arcs extending from an apex of the dome to the base portion of the dome and teeth positioned in the apertures.

Document US-A-2007/0142840 discloses an instrument assembly for use in orthopaedic surgery comprising a reamer to be positioned within a body cavity to engage a bone, having one bar portion extending across it.

One purpose of the present invention is to achieve a milling tool for prosthetic surgery operations that is extremely precise and stable in operations.

Another purpose is to achieve a milling tool that reduces to a minimum the risk of jamming and that is not affected by the different hardnesses of the tissue, avoiding damage to the bone.

The Applicant has devised, tested and embodied the present invention to overcome the shortcomings of the state of the art and to obtain these and other purposes and advantages.

### SUMMARY OF THE INVENTION

The present invention is set forth and characterized in the independent claim, while the dependent claims describe other characteristics of the invention or variants to the main inventive idea.

In accordance with the above purposes, a milling tool for prosthetic surgery operations according to the present invention comprises a cap body, hollow inside, at least a cutting profile associated with the cap body and an attachment support positioned and constrained with respect to the cap body and by means of which the cap body is selectively associable with a relative manual or automatic manipulator.

The embodiment shown in Figs. 4-6 forms the basis for the two-part form of claim 1. The embodiment shown in Figs. 4-6 forms the basis for the two-part form of claim 1. The embodiment shown in Figs. 4-6 forms the basis for the two-part form of claim 1. The embodiment shown in Figs. 4-6 forms the basis for the two-part form of claim 1. The embodiment shown in Figs. 4-6 forms the basis for the two-part form of claim 1. The embodiment shown in Figs. 4-6 forms the basis for the two-part form of claim 1. The embodiment shown in Figs. 4-6 forms the basis for the two-part form of claim 1. The embodiment shown in Figs. 4-6 forms the basis for the two-part form of claim 1. The embodiment shown in Figs. 4-6 forms the basis for the two-part form of claim 1. The embodiment shown in Figs. 4-6 forms the basis for the two-part form of claim 1.

According to one feature of the present invention, the attachment support comprises a diametral pin having a length substantially equivalent to the diameter of a lower circular edge of the cap body and having the respective ends positioned and constrained to the lower circular edge. Moreover, the attachment support comprises a connection cylinder attached by means of an attachment pin of the same attachment support to a median zone of the diametral pin, so as to be in a position substantially coaxial with a median axis of rotation of the cap body.

This solution has the advantage that it allows a perfectly symmetrical and balanced rotation of the cap body, performing an effective and precise action of cutting and incising the surfaces concerned.

According to one form of embodiment of the present invention, on the hollow cap body at least a through aperture is made, with an oblong conformation, on at least one edge of which the cutting profile is made.

In some forms of embodiment the cutting profile substantially concerns the whole length of the edge of the through aperture.

According to another characteristic feature of the present invention, the through aperture starts from a polar zone of the cap body and extends toward a lower edge of the cap body.

According to another characteristic feature of the present invention, the ratio between the length of the through aperture and the diameter of the cap body has a value comprised between about 0.6 and about 0.8.

In this way, the milling tool comprises at least one through aperture and at least one cutting profile, distributed and oriented along the circular flank of the cap body, so as to improve the conditions for discharging the tissue removed by the cutting profile.

With the present invention therefore, given the same overall length of the cutting profile provided over the whole cap body, compared with known solutions, the distribution and length provided for each through aperture and for each cutting profile allow not only a uniform milling operation but also to optimize the cutting and discharge conditions of the tissue, so as to reduce to a minimum the risk of blockages and hence operating interruptions to the tool.

In fact, the conformation and disposition of the through apertures is sufficient to allow the tissue progressively excavated to be discharged from the tool through the thickness of the cap body without depositing in the aperture and therefore without blocking it. Therefore, the solution according to the present invention reduces to a minimum the risk of losses of efficiency and quality of the operations performed.

This solution provides cutting profiles of substantially prevalent sizes on the edge of the cap body, which can incise simultaneously both hard tissues, like the bones, and also soft tissues, like the ligaments.

This solution therefore allows to reduce to a minimum the risk of damaging the bone structure during the removal steps.

According to a variant, a plurality of through apertures are provided with an oblong conformation, each provided with a relative cutting profile, and angularly offset with respect to each other on the surface of the cap body, in order to improve the effectiveness of the operating action with a great structural rigidity of the cap body.

In some forms of embodiment, the through apertures are comprised between 2 and 4.

According to another variant, each through aperture is offset with respect to the polar zone of the cap body, so as to cover substantially the whole operating extension of the cap body.

According to another variant, the cutting profile of each cap body is conformed in an indented manner, so as to define a plurality of breaks in continuity, able to promote the breakage of the excavated parts and hence to facilitate the discharge thereof.

According to another variant, each through aperture is inclined by a determinate angle with respect to a median axis of rotation of the cap body, so as to define a desired axial angle of the cutting profile.

With this solution, the tool's incision and cutting conditions are further improved, with a consequent improvement in quality of the work carried out.

According to another form of embodiment, a plurality of through holes are made on the cap body, each provided with a cutting edge, so as to define overall the cutting profile to perform a mechanical action of excavation on the bone.

### BRIEF DESCRIPTION OF THE DRAWINGS

These and other characteristics of the present invention will become apparent from the following description of a preferential form of embodiment, given as a non-restrictive example with reference to the attached drawings wherein:
- fig. 1 is a three-dimensional view of a tool according to the present invention;
- fig. 2 is a plane view of the tool in fig. 1;
- fig. 3 is a view in section from II to II of fig. 2;
- fig. 4 is an enlarged detail of the section in fig. 3;
- fig. 5 is a three-dimensional view of an embodiment of the present invention;
- fig. 6 is a section view of fig. 5

To facilitate comprehension, the same reference numbers have been used, where possible, to identify identical common elements in the drawings.

### DETAILED DESCRIPTION OF SOME FORMS OF EMBODIMENT

With reference to the attached drawings, the reference number 10 is used to denote in its entirety a milling tool according to the present invention, used in prosthetic surgery operations, in this case to achieve the bone seating for the cup of an acetabular prosthesis of the hip.

In particular, the tool 10 comprises a cap body 11, in this case semi-spherical, and an attachment support 12 by means of which the tool 10 can be selectively associated with a relative manual or automatic manipulator, not shown in the drawings.

The cap body 11 is substantially hollow inside and comprises a plurality of through apertures 13, in this case four, disposed angularly offset with respect to each other.

Each through aperture 13 has a substantially oblong conformation with a ratio between its length and the diameter of the cap body 11 comprised between about 0.6 and about 0.8, and extends from a polar zone 15 of the cap body 11 to a lower circular edge 16 of the cap body 11.

To give an example, the through apertures 13 have a length varying from about 20mm to about 55mm, while the cap body 11 has a diameter comprised between about 30mm and about 80mm.

Moreover, each through aperture 13 has an inclination of a determinate angle α comprised between about 30° and about 40°, with respect to an ideal median axis of rotation X of the cap body 11, passing through the polar zone 15.

Consequently, the apertures 13 have a curvature on two Cartesian planes, that is, as well as following the curvature of the cap body 11 they are also arched on the length, defining a saber-shaped conformation.

In particular, the convexity of the curvature is concordant with the direction of rotation.

Furthermore, the four through apertures 13 are offset to each other with respect to the polar zone 15, in this case, two close and two distanced, so as to cover substantially the whole external surface of the cap body 11, and in particular the operating shape of the latter when made to rotate.

Each through aperture 13 defines, along one edge, a relative cutting profile 17 which extends substantially for the whole length of the edge, thus affecting the whole through aperture 13.

In particular, each cutting profile 17 not only is suitably sharp to cut tissues and bone structures, but also is inclined toward the outside of the external circular surface of the cap body 11 so as to define a relative hook angle β comprised between about 40° and about 50°, and a relative rake angle δ comprised between about 15° and about 25°.

Advantageously, the cutting profile 17 faces in a direction concordant with the direction of rotation of the cap body 11.

By exploiting the angle of inclination α of the through aperture 13 with respect to the median axis of rotation of the cap body 11, the cutting profile 17 also defines an axial angle, substantially coinciding with the angle α.

Each cutting profile 17 also comprises a plurality of semi-holes 19, equidistant from each other and converging toward the center, able to define an indented development of the cutting profile 17. In this way, each cutting profile 17 defines relative breaks in continuity on its length, so as to promote the breakage of the tissue once cut, and hence to facilitate the discharge thereof through the relative through aperture 13.

Advantageously, each cutting profile 17 has a length varying between about 2mm and about 5mm.

Along the lower circular edge 16 of the cap body 11, in diametrically opposite positions, two positioning grooves 20 are provided, suitable for positioning and constraining the attachment support 12.

The attachment support 12 comprises a diametral pin 21, or cylinder or suchlike, or comparable connection element 22 for connection to a manipulator, and a pin or equivalent attachment element 23.

The diametral pin 21 has a length substantially equivalent to the diameter of the lower circular edge 16 of the cap body 11, and has its respective ends positioned and constrained, for example by welding, to the positioning grooves 20, so as to connect the attachment support 12 structurally to the cap body 11.

Furthermore, the diametral pin 21 in this case has a constraint seating 21a for the pin 23, which is made in the median zone of the diametral pin 21 and aligned during use with the median axis of rotation X of the cap body 11.

The connection cylinder 22 also has a first through hole 22a, made in a direction orthogonal to the median axis of rotation X, configured for the stable insertion of the diametral pin 21.

Furthermore, the connection cylinder 22 is attached by means of the pin 23 to the median zone of the diametral pin 21, so as to be in a position substantially coaxial to the median axis of rotation of the cap body 11. In this way, when the tool is associated with a relative manual or automatic manipulator, in order to actuate the operating rotation thereof, the rotation is perfectly symmetrical and balanced, performing an effective and precise action of cutting and incision on the surfaces concerned.

In particular, in the solution shown, the connection cylinder 22 has a second through hole 22b, which intersects the first hole 22a and which is aligned in use with the median axis of rotation X of the cap body 11, and hence with the constraining seating 21a of the diametral pin. In this way, by inserting the pin 23 through the second hole 22b and into the constraining seating 21a, a stable attachment is obtained between the connection cylinder 22 and the diametral pin 21.

It is clear that modifications and/or additions of parts may be made to the milling tool as described heretofore, without departing from the field and scope of the present invention.

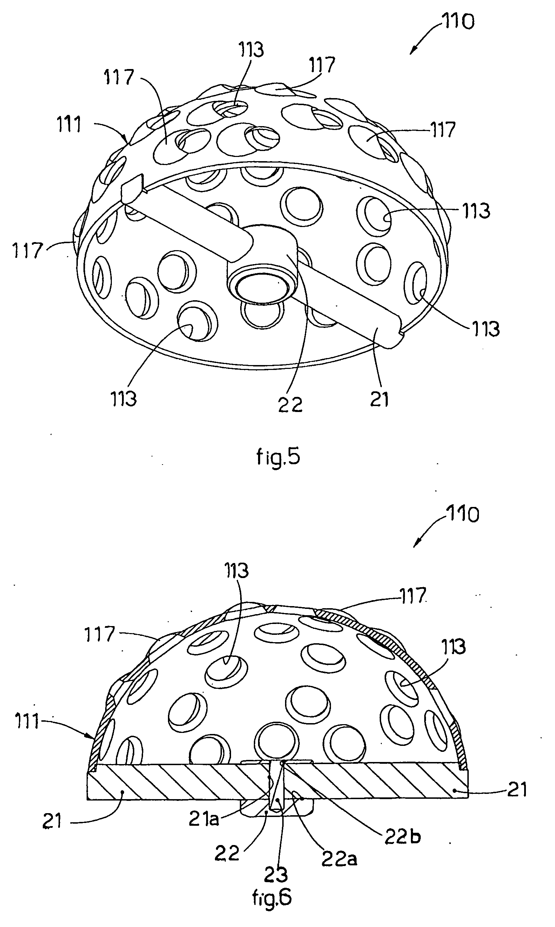

For example, figs. 5 and 6 show another form of embodiment of the present invention indicated for convenience by the reference number 110, where the same reference numbers are used for parts identical to those in the previous form of embodiment. In this form of embodiment, the cap body, indicated for convenience by the reference number 111, has a plurality of through holes 113 each provided with a cutting edge or profile 117, and distributed uniformly and homogeneously along the surface so as to define an overall cutting profile to perform the mechanical action of excavating the bone. This solution in practice achieves a "grater" conformation of the cutting profile, to perform the operations of incision and removal of the bone material.

It is also clear that, although the present invention has been described with reference to some specific examples, a person of skill in the art shall certainly be able to achieve many other equivalent forms of milling tool for prosthetic surgery operations, having the characteristics as set forth in the claims and hence all coming within the field of protection defined thereby.

## Claims

1. Milling tool for prosthetic surgery operations comprising a cap body (11, 111), hollow inside, and at least a cutting profile (17), associated with said cap body (11, 111), an attachment support (12) positioned and constrained with respect to the cap body (11, 111) and by means of which said cap body (11, 111) can be selectively associated with a relative manual or automatic manipulator, wherein the attachment support (12) comprises a diametral pin (21), a connection cylinder (22), and an attachment pin (23), said diametral pin (21) having a length substantially equivalent to the diameter of a lower circular edge (16) of the cap body (11, 111) and having the respective ends positioned and constrained to said lower circular edge (16), **characterized in that** the diametral pin has a constraining seating (21 a) for the attachment pin (23), the connection cylinder (22) has a through hole (22b) which is aligned in use with the constraining seating (21a), and the connection cylinder (22) is attached by means of the attachment pin (23) to a median zone of the diametral pin (21) and aligned with a median axis of rotation (X) of the cap body (11, 111), so as to be in a position substantially coaxial to said median axis of rotation (X) of the cap body (11, 111).

2. Tool as in claim 1, **characterized in that** on said cap body (11) at least a through aperture (13) is made, with an oblong conformation, on at least one edge of which said cutting profile (17) is made, said through aperture (13) starting from a polar zone (15) of said cap body (11) and extending toward a lower edge The embodiment shown in Figs. 4-6 forms the basis for the two-part form of claim 1. (16) of said cap body (11) with a ratio between the length of the through aperture and the diameter of said cap body (11) having a value comprised between about 0.6 and about 0.8.

3. Tool as in claim 2, **characterized in that** it comprises a plurality of through apertures (13), each provided with a relative cutting profile (17) and angularly offset with respect to each other on the surface of the cap body (11).

4. Tool as in claim 3, **characterized in that** each through aperture (13) is offset with respect to the polar zone (15) of the cap body (11).

5. Tool as in any claim from 2 to 4, **characterized in that** each through aperture (13) is inclined by a determinate angle (α) with respect to a median axis of rotation (X) of the cap body (11).

6. Tool as in claim 5, **characterized in that** the determinate angle (α) of inclination of each through aperture (13) is comprised between about 30° and about 40°.

7. Tool as in any claim hereinbefore, **characterized in that** the cutting profile (17) comprises a plurality of semi-holes (19) equidistant with respect to each other so as to define a plurality of breaks in continuity in said cutting profile (17),

8. Tool as in any claim hereinbefore, **characterized in that** the cutting profile (17) is inclined toward the outside of an external circular surface of the cap body (11), so as to define at least a relative hook angle (β) and a relative rake angle (δ).

9. Tool as in claim 8, **characterized in that** the hook angle (β) is comprised between about 40° and about 50°.

10. Tool as in claim 8 or 9, **characterized in that** the rake angle (δ) is comprised between about 15° and about 25°.

11. Tool as in claim 1, **characterized in that** a plurality of through holes (113) are made on said cap body (111), each provided with a cutting edge (117), so as to define all in all the cutting profile.

12. Tool as in any claim hereinbefore, **characterized in that** the constraint seating (21a) is in the median zone of the diametral pin (21) and aligned during use with the median axis of rotation (X) of the cap body (11). The embodiment shown in Figs. 4-6 forms the basis for the two-part form of claim 1. The embodiment shown in Figs. 4-6 forms the basis for the two-part form of claim 1.

13. Tool as in any claim hereinbefore, **characterized in that** the connection cylinder (22) has a first through hole (22a) and a second through hole (22b), wherein the first through hole (22a) is made in a direction orthogonal to the median axis of rotation (X), configured for the stable insertion of the diametral pin (21), and the second through hole (22) is the through hole (22b) that is aligned in use with the constraining seating (21a).

14. Tool as in claim 13, **characterized in that** the second through hole (22b) The embodiment shown in Figs. 4-6 forms the basis for the two-part form of claim 1. intersects the first through hole (22a) and is aligned with the median axis of rotation (X) of the cap body (11, 111), and hence with the constraining seating (21a) of the diametral pin (21).

## Patentansprüche

1. Fräswerkzeug für chirurgische Protheseoperationen, welches einen Aufsatzkörper (11, 111), welcher im Inneren ausgehöhlt ist, und zumindest ein Schneidprofil (17), welches mit dem Aufsatzkörper (11, 111) in Zusammenhang steht, eine Befestigungshalterung (12), welche mit Bezug auf den Aufsatzkörper (11, 111) positioniert und gehalten ist, und mittels derer der Aufsatzkörper (11, 111) mit einer jeweiligen manuellen oder automatischen Manipulationseinrichtung selektiv in Verbindung gebracht werden kann, enthält, wobei die Befestigungshalterung (12) einen diametralen Stift (21), einen Verbindungszylinder (22) und einen Befestigungsstift (23) enthält, wobei der diametrale Stift (21) eine Länge hat, welche im Wesentlichen gleich dem Durchmesser einer unteren kreisförmigen Kante (16) des Aufsatzkörpers (11, 111) ist, und jeweilige Enden hat, welche zu der unteren kreisförmigen Kante (16) positioniert und daran gehalten sind, **dadurch gekennzeichnet, dass** der diametrale Stift einen Haltesitz (21a) für den Befestigungsstift (23) hat, der Verbindungszylinder (22) ein Durchgangsloch (22b) hat, welches im Gebrauch zum Haltesitz (21a) ausgerichtet ist, und der Verbindungszylinder (22) mittels des Befestigungsstifts (23) an einem Mittelbereich des diametralen Stifts (21) befestigt ist und zu einer mittleren Drehachse (X) des Aufsatzkörpers (11, 111) ausgerichtet ist, um somit in einer Position zu sein, welche im Wesentlichen koaxial zur mittleren Drehachse (X) des Aufsatzkörpers (11, 111) ist.

2. Werkzeug nach Anspruch 1, **dadurch gekennzeichnet, dass** auf dem Aufsatzkörper (11) zumindest eine Durchgangsöffnung (13) mit einer langgestreckten Ausbildung erstellt ist, wobei auf zumindest einer Kante davon das Schneidprofil (17) erstellt ist, wobei die Durchgangsöffnung (13) von einer Polarzone (15) des Aufsatzkörpers (11) beginnt und sich in Richtung zu einer unteren Kante (16) des Aufsatzkörpers (11) erstreckt, wobei ein Verhältnis zwischen der Länge der Durchgangsöffnung und dem Durchmesser des Aufsatzkörpers (11) einen Wert hat, welcher zwischen ungefähr 0,6 und ungefähr 0,8 beträgt.

3. Werkzeug nach Anspruch 2, **dadurch gekennzeichnet, dass** es eine Mehrzahl von Durchgangsöffnungen (13) enthält, wobei jede mit einem relativen Schneidprofil (17) bereitgestellt ist, und welche auf der Fläche des Aufsatzkörpers (11) zueinander winkelförmig versetzt sind.

4. Werkzeug nach Anspruch 3, **dadurch gekennzeichnet, dass** jede Durchgangsöffnung (13) mit Bezug zur Polarzone (15) des Aufsatzkörpers (11) versetzt ist.

5. Werkzeug nach einem der Ansprüche 2 bis 4, **dadurch gekennzeichnet, dass** jede Durchgangsöffnung (13) mit einem bestimmten Winkel (α) mit Bezug auf eine mittlere Drehachse (X) des Aufsatzkörpers (11) geneigt ist.

6. Werkzeug nach Anspruch 5, **dadurch gekennzeichnet, dass** der bestimmte Winkel (α) der Neigung von jeder Durchgangsöffnung (13) zwischen ungefähr 30° und ungefähr 40° beträgt.

7. Werkzeug nach einem der vorhergehenden Ansprüche, **dadurch gekennzeichnet, dass** das Schneidprofil (17) eine Mehrzahl von Halblöchern (19) enthält, welche zueinander gleich weit beabstandet sind, um somit eine Mehrzahl von Unterbrechungen in der Kontinuität im Schneidprofil (17) zu bestimmen.

8. Werkzeug nach einem der vorhergehenden Ansprüche, **dadurch gekennzeichnet, dass** das Schneidprofil (17) in Richtung zur Außenseite einer externen kreisförmigen Fläche des Aufsatzkörpers (11) geneigt ist, um somit zumindest einen jeweiligen Radialwinkel (β) und einen jeweiligen Spannwinkel (δ) zu bestimmen.

9. Werkzeug nach Anspruch 8, **dadurch gekennzeichnet, dass** der Radialwinkel (β) zwischen ungefähr 40° und ungefähr 50° beträgt.

10. Werkzeug nach Anspruch 8 oder 9, **dadurch gekennzeichnet, dass** der Spannwinkel (δ) zwischen ungefähr 15° und ungefähr 25° beträgt.

11. Werkzeug nach Anspruch 1, **dadurch gekennzeichnet, dass** eine Mehrzahl von Durchgangslöchern (113) auf dem Aufsatzkörper (111) erstellt sind, wobei jedes mit einer Schneidkante (117) bereitgestellt ist, um somit insgesamt das Schneidprofil zu bestimmen.

12. Werkzeug nach einem der vorhergehenden Ansprüche, **dadurch gekennzeichnet, dass** der Haltesitz (21a) im mittleren Bereich des diametralen Stifts (21) erstellt ist und im Gebrauch zur mittleren Drehachse (X) des Aufsatzkörpers (11) ausgerichtet ist.

13. Werkzeug nach einem der vorhergehenden Ansprüche, **dadurch gekennzeichnet, dass** der Verbindungszylinder (22) ein erstes Durchgangsloch (22a) und ein zweites Durchgangsloch (22b) hat, wobei das erste Durchgangsloch (22a) in einer Richtung senkrecht zur mittleren Drehachse (X) erstellt ist, ausgelegt zum dauerhaften Einsetzen des diametralen Stifts (21), und wobei das zweite Durchgangsloch (22b) jenes Durchgangsloch (22b) ist, welches im Gebrauch zum Haltesitz (21a) ausgerichtet ist.

14. Werkzeug nach Anspruch 13, **dadurch gekennzeichnet, dass** das zweite Durchgangsloch (22b) das erste Durchgangsloch (22a) kreuzt, und zur mittleren Drehachse (X) des Aufsatzkörpers (11, 111) und somit zum Haltesitz (21a) des diametralen Stifts (21) ausgerichtet ist.

## Revendications

1. outil de fraisage pour des opérations chirurgicales prothétiques comprenant un corps de capuchon (11, 111), un intérieur creux et au moins un profil de coupe (17) associé audit corps de capuchon (11, 111), un support de fixation (12) placé et contraint par rapport au corps de capuchon (11, 111) et au moyen duquel ledit corps de capuchon (11, 111) peut être associé sélectivement à un manipulateur manuel relatif ou automatique, dans lequel le support de fixation (12) comprend une broche diamétrale (21), un cylindre de connexion (22) et une broche de fixation (23), ladite broche diamétrale (21) ayant une longueur substantiellement équivalente au diamètre d'un bord circulaire inférieur (16) du corps de capuchon (11, 111) et ayant les extrémités respectives placées et contraintes par rapport audit bord circulaire inférieur (16), **caractérisé en ce que** la broche diamétrale a une assise contraignante (21a) pour la broche de fixation (23), le cylindre de connexion (22) a un trou débouchant (22b) qui est aligné lors de l'utilisation avec l'assise contraignante (21a) et le cylindre de connexion (22) est fixé au moyen de la broche de fixation (23) à une zone médiane de la broche diamétrale (21) et aligné avec un axe de rotation médian (X) du corps de capuchon (11, 111) de façon à être dans une position substantiellement coaxiale avec ledit axe de rotation médian (X) du corps de capuchon (11, 111).

2. Outil selon la revendication 1, **caractérisé en ce que** sur ledit corps de capuchon (11), au moins une ouverture débouchante (13) est pratiquée, de conformation oblongue, sur au moins un bord duquel ledit profil de coupe (17) est réalisé, ladite ouverture débouchante (13) commençant par une zone polaire (15) dudit corps de capuchon (11) et s'étendant vers un bord inférieur (16) dudit corps de capuchon (11) avec un rapport entre la longueur de l'ouverture débouchante et le diamètre dudit corps de capuchon (11) ayant une valeur comprise entre environ 0,6 et environ 0,8.

3. Outil selon la revendication 2, **caractérisé en ce qu'**il comprend une pluralité de trous débouchants (13), chacun étant fourni avec un profil de coupe relatif (17) et un décalage angulaire les uns par rapport aux autres sur la surface du corps de capuchon (11).

4. Outil selon la revendication 3, **caractérisé en ce que** chaque ouverture débouchante (13) est décalée par rapport à la zone polaire (15) du corps de capuchon (11).

5. Outil selon l'une quelconque des revendications 2 à 4, **caractérisé en ce que** chaque ouverture débouchante (13) est inclinée d'un angle déterminé (α) par rapport à un axe de rotation médian (X) sur le corps de capuchon (11).

6. Outil selon la revendication 5, **caractérisé en ce que** l'angle déterminé (α) d'inclinaison de chaque ouverture débouchante (13) est compris entre environ 30° et environ 40°.

7. Outil selon l'une quelconque des revendications précédentes, **caractérisé en ce que** le profil de coupe (17) comprend une pluralité de semi-alésages (19) équidistants les uns des autres de façon à définir une pluralité de ruptures de continuité dans ledit profil de coupe (17).

8. Outil selon l'une quelconque des revendications précédentes, **caractérisé en ce que** le profil de coupe (17) est incliné vers l'extérieur d'une surface circulaire externe du corps de capuchon (11) de façon à définir au moins un angle d'attaque relatif (β) et un angle incliné (δ) relatif.

9. Outil selon la revendication 8, **caractérisé en ce que** l'angle d'attaque (β) est compris entre environ 40° et environ 50°.

10. Outil selon la revendication 8 ou 9, **caractérisé en ce que** l'angle incliné (δ) est compris entre environ 15° et environ 25°.

11. Outil selon la revendication 1, **caractérisé en ce qu'**une pluralité de trous débouchants (113) sont pratiqués sur ledit corps de capuchon (111), chacun étant doté d'un bord coupant (117) de façon à définir dans l'ensemble le profil de coupe.

12. Outil selon l'une quelconque des revendications précédentes, **caractérisé en ce que** l'assise contraignante (21a) est produite dans la zone médiane de la broche diamétrale (21) et alignée pendant l'utilisation avec l'axe de rotation médian (X) du corps de capuchon (11).

13. Outil selon l'une quelconque des revendications précédentes, **caractérisé en ce que** le cylindre de connexion (22) a un premier trou débouchant (22a) et un deuxième trou débouchant (22b), le premier trou débouchant (22a) étant produit dans une direction orthogonale par rapport à l'axe de rotation médian (X), configuré pour l'insertion stable de la broche diamétrale (21), et le deuxième trou débouchant (22b) étant le trou débouchant (22b) qui est aligné lors de l'utilisation avec l'assise contraignante (21a).

14. Outil selon la revendication 13, **caractérisé en ce que** le deuxième trou débouchant (22b) croise le premier trou débouchant (22a) et est aligné avec l'axe de rotation médian (X) du corps de capuchon (11, 111) et donc avec l'assise contraignante (21a) de la broche diamétrale (21).
